# EUROPEAN PATENT APPLICATION

(11) **EP 1 988 481 A2**
(43) Date of publication of application: **05.11.2008**
(21) Application number: 08008329.8
(22) Date of filing: 02.05.2008
(51) Int. Cl.: G06F 19/00, G06F 3/00

(54) **System and method of providing patient information**

(30) Priority: 04.05.2007 KR 20070043490
(71) Applicant: MEDISON CO., LTD., Kangwon-do 250-870 (KR)
(72) Inventor: Nam, Sang Gyu, Gangnam-gu Seoul 135-280 (KR)
(74) Representative: Schmid, Wolfgang

(57) **Abstract**

The present invention relates to an ultrasound system. The present invention provides a system of providing patient information in the ultrasound system. The system comprises: a database to store patient information including basic information and additional information of at least one patient; an input unit operable to receive a user input in a syllable unit or a word unit to produce at least one syllable or word; a processor operable to retrieve the basic information matched with the at least one syllable or word from the database and form a candidate list of the basic information based on the retrieved basic information; and a display unit operable to display the candidate list of the basic information.

## Description

The present application claims priority from Korean Patent Application No. 10-2007-0043490 filed on May 4, 2007, the entire subject matter of which is incorporated herein by reference.

### BACKGROUND OF THE INVENTION

### [Technical Field]

The present invention generally relates to ultrasound systems, and more particularly to a system and a method of providing patient information.

### [Background Art]

An ultrasound system has become an important and popular diagnostic tool due to its non-invasive and non-destructive nature. Modern high-performance ultrasound imaging diagnostic systems and techniques are commonly used to produce two- or three-dimensional images of internal features of patients.

The ultrasound system generally uses a probe containing an array of piezoelectric elements to transmit and receive ultrasound signals. The ultrasound system forms an image of human internal tissues by electrically exciting transducer elements to generate ultrasound signals that travel into the body. Echoes reflected from tissues and organs return to the transducer element and are converted into electrical signals, which are amplified and processed to produce ultrasound data. The ultrasound data may include volume data obtained by using a 3-dimensional probe, etc.

Generally, the ultrasound system receives patient information before scanning a specific part of a patient. The patient information may include basic information such as an identification (ID), name, date of birth, etc. of a patient and additional information depending on medical departments. The patient information may be stored in a database. Also, the ultrasound system may provide the patient information stored in the database based on the ID, name or date of birth of the patient.

According to the conventional ultrasound system, the patient information may be provided for display only after the entire syllables of the basic information of the patient have been completely inputted by the user. In such a case, incorrect patient information may be provided due to an incorrect input of the ID or name of the corresponding patient.

### BRIEF DESCRIPTION OF THE DRAWINGS

FIG. 1 is a block diagram showing a system of providing patient information in accordance with one embodiment of the present invention.

FIG. 2 is a schematic diagram showing an example of a basic information input window in accordance with one embodiment of the present invention.

FIG. 3 is a diagram showing an example of displaying a first candidate list of basic information in accordance with one embodiment of the present invention.

FIG. 4 is a diagram showing an example of displaying a second candidate list of basic information in accordance with one embodiment of the present invention.

FIG. 5 is a schematic diagram showing an example of displaying an additional information providing window in accordance with one embodiment of the present invention.

FIG. 6 is a schematic diagram showing an example of displaying a third candidate list of basic information in accordance with another embodiment of the present invention.

FIG. 7 is a schematic diagram showing an example of displaying a fourth candidate list of basic information in accordance with another embodiment of the present invention.

FIG. 8 is a schematic diagram showing an example of displaying a basic information providing window in accordance with another embodiment of the present invention.

FIG. 9 is a schematic diagram showing an example of displaying an additional information providing window in accordance with another embodiment of the present invention.

### DETAILED DESCRIPTION OF THE INVENTION

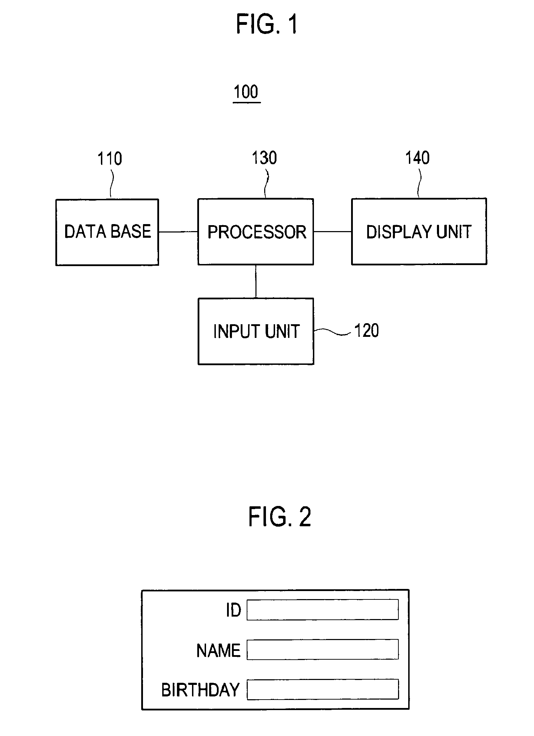

FIG. 1 is a block diagram showing a patient information providing system in accordance with present invention. The patient information providing system may include a database 110, an input unit 120, a processor 130 and a display unit 140.

The database 110 may store patient information including basic information of a patient and additional information. The items of the basic information may include identification (ID), name and date of birth of the patient without limitation. The items of the additional information may include a medical department, a physical condition, a disease record, a medical record, a doctor, a patient photo, etc. The database 110 may store a plurality of ultrasound images corresponding to the respective patients. It should be understood that numerous other modifications of the items of the patient information including the basic information and the additional information can be devised by those skilled in the art.

The input unit 120 may receive a user's input in a syllable unit or word unit. More particularly, the input unit 120 may allow the user to input the respective items of the basic information in a syllable unit or a word unit. The input unit 120 may further allow the user to input a selection instruction for selecting one of the items of the basic information from a candidate list of the basic information, which is displayed on the display unit 140. The input unit 120 may be one or more of a mouse, a keyboard and a trackball.

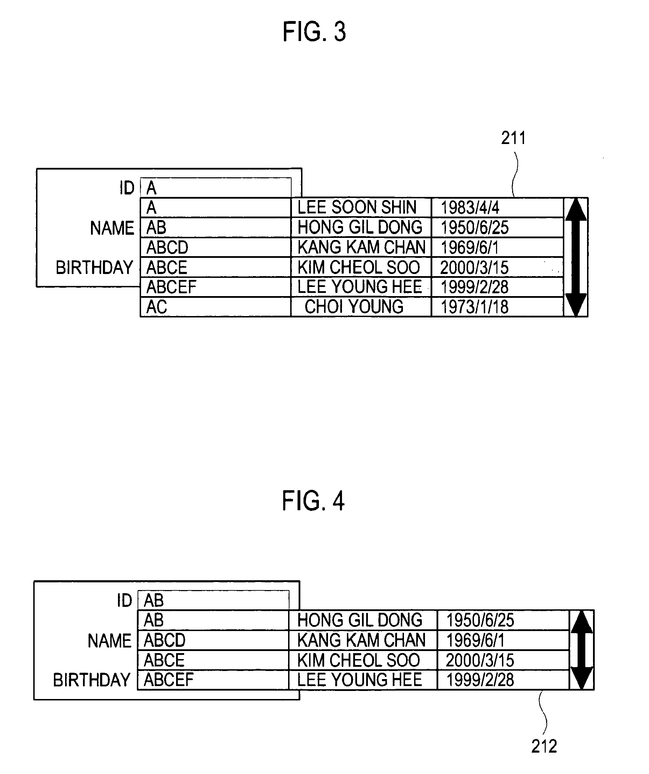

The processor 130 may be operable to form a basic information input window for allowing the user to input the items of the basic information through the input unit 120, as illustrated in FIG. 2. The processor 130 may be operable to retrieve basic information including the syllables or words of the item of the basic information, which is inputted through the input unit 120 by the user, from the database 110. The processor 130 may be operable to form a candidate list of the basic information based on the retrieved basic information. For example, if the processor 130 receives an input of a first syllable "A" of the ID among the items of the basic information as illustrated in FIG. 3, then the processor 130 may be operable to retrieve the basic information corresponding to the Ids, the first syllable of which is "A" from the database 110. The processor 130 may be operable to form a first candidate list 211 of the basic information based on the retrieved basic information.

Subsequently, the processor 130 may check whether a second syllable of the ID in the basic information has been inputted through the input unit 120 or whether one of IDs has been selected from the first candidate list of the basic information. If it is determined that the second syllable of the ID in the basic information has been inputted through the input unit 120, then the processor 130 may retrieve basic information, including the IDs whose second syllable is identical to the inputted second syllable, e.g., "B" from the first candidate list of the basic information. The processor 130 may be operable to form a second candidate list of the basic information based on the retrieved basic information. The processor 130 may be operable to form the second candidate list of the basic information, which includes the names and dates of birth of the corresponding IDs, as illustrated in FIG. 4.

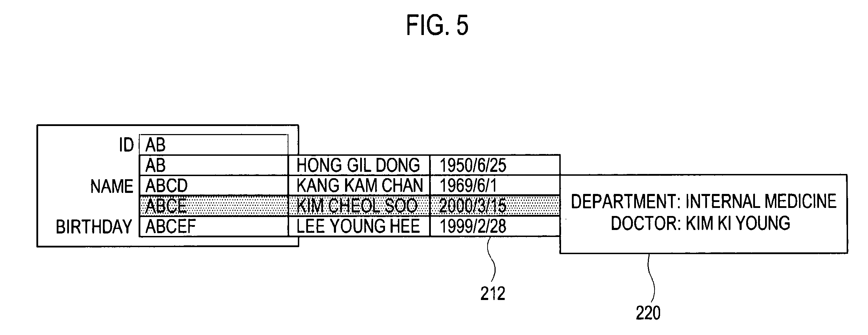

The processor 130 may check whether a third syllable of the basic information has been inputted through the input unit 120 or whether one of IDs has been selected from the second candidate list of the basic information. If it is determined that one of the IDs has been selected from the second candidate list of the basic information through the input unit 120, then the processor 130 may be operable to retrieve additional information corresponding to the selected ID from the database 110. The processor 130 may be operable to form an additional information providing window 220 showing the retrieved addition information, as illustrated in FIG. 5. Although a medical department and a doctor are provided as additional information on the additional information providing window 220 as shown in FIG. 5, the additional information is certainly not limited thereto. It should be understood that a patient photo, a physical condition and a disease record may be provided on the additional information providing window 220 by those skilled in the art.

In accordance with another embodiment of the present invention, the processor 130 may receive a first syllable of one of the items including ID, name and date of birth of the basic information through the input unit 120. Hereinafter, for the sake of convenience of explanation, it is assumed that a first syllable of ID is inputted through the input unit 120 from the user.

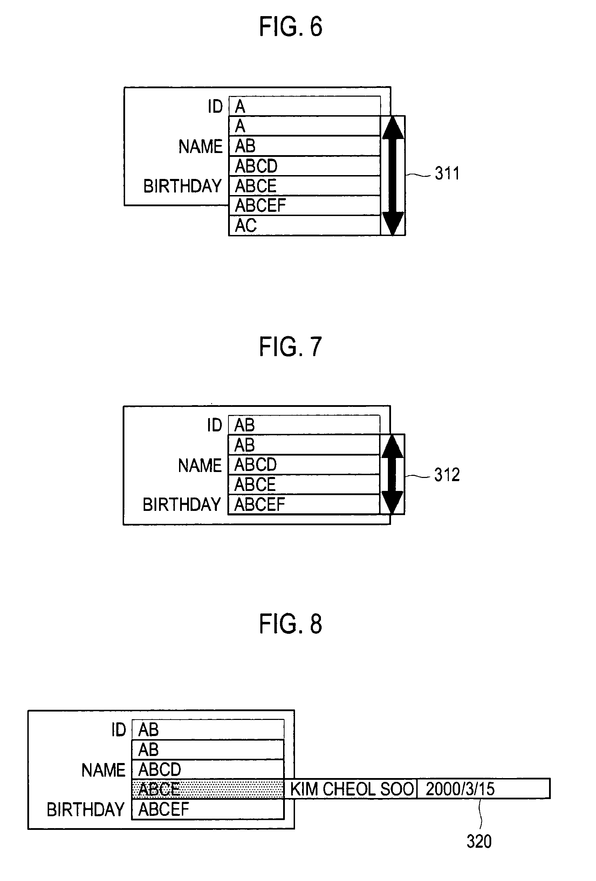

The processor 130 may be operable to retrieve basic information including IDs whose first syllable is identical to the inputted syllable "A" from the database 110 and form a third candidate list 311 of the basic information based on the retrieved basic information as illustrated in FIG. 6. Thereafter, the processor 130 may be operable to check whether a second syllable of the ID has been inputted through the input unit 120 or whether one of IDs has been selected from the third candidate list 311 of the basic information. If it is determined that the second syllable of the ID in the basic information has been inputted through the input unit 120, then the processor 130 may be operable to retrieve basic information including IDs whose second syllable is identical to the inputted second syllable "B" from the third candidate list 311 of the basic information. The processor 130 may be operable to form a fourth candidate list 312 of the basic information based on the retrieved basic information, as illustrated in FIG. 7.

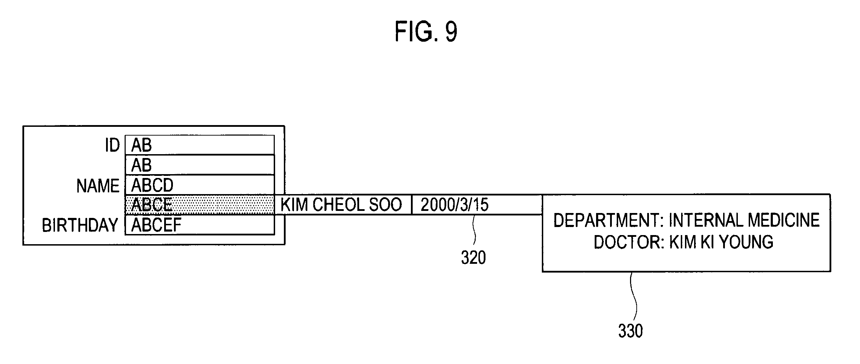

Subsequently, the processor 130 may be operable to check whether a third syllable is inputted through the input unit 120 or whether one of the IDs is selected from the fourth candidate list 312 of the basic information. If it is determined that one of the IDs is selected from the fourth candidate list 312 of the basic information through the input unit 120, then the processor 130 may be operable to retrieve basic information corresponding to the selected ID from the database 110. The processor 130 may be operable to form a basic information providing window 320 showing the retrieved basic information, as illustrated in FIG. 8. Further, the processor 130 may be operable to retrieve additional information corresponding to the selected ID from the database 110 and form an additional information providing window 330 showing the retrieved additional information, as illustrated in FIG. 9.

Although the processor 130 may provide the patient information by receiving the basic information of the patient in a syllable unit through the input unit 120 in accordance with above-mentioned embodiments, the processor 130 may provide the patient information by receiving information of the patient in a word unit.

The display unit 140 may be operable to display the basic information input window, the candidate list of the basic information, the basic information providing window and the additional information providing window.

As mentioned above, the present invention can provide the patient information based on the syllables or words inputted through the input unit. Thus, even if the similar IDs or names may exist in the database, accurate patient information can be selected.

In accordance with one aspect of the present invention, there is provided a system of providing patient information, comprising: a database to store patient information including basic information including and additional information of at least one patient; an input unit operable to receive a user input in a syllable unit or a word unit to produce at least one syllable or word; a processor operable to retrieve the basic information matched with the at least one syllable or word from the database and form a candidate list of the basic information based on the retrieved basic information; and a display unit operable to display the candidate list of the basic information.

In accordance with another aspect of the present invention, there is provided a method of providing patient information, comprising: preparing database of patient information including basic information and additional information of patients; receiving the basic information in a syllable unit or a word unit from a user; retrieving basic information including the received syllable or word from the database; and providing a candidate list of the basic information including the retrieved basic information.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A system for providing patient information, comprising:
a database operable to store patient information including basic information and additional information of at least one patient;
an input unit operable to receive a user input in a syllable unit or a word unit to produce at least one syllable or word;
a processor operable to receive the basic information matched with the at least one syllable or word from the database and forming a candidate list of the basic information based on the retrieved basic information; and
a display unit operable to display the candidate list of the basic information.

2. The system of Claim 1, wherein the basic information includes ID, name and date of birth of the patient, and wherein the additional information includes a medical department, a physical condition, a disease record, a medical record, a doctor and a photo of the patient.

3. The system of Claim 2, wherein the processor forms an input window on the display unit for allowing the user to input the user input and retrieves the basic information including the syllable or word inputted in the input window from the database, the processor being configured to form the candidate list of the basic information based on the retrieved basic information.

4. The system of Claim 3, wherein the basic information inputted to the input window is one of ID, name and date of birth.

5. The system of Claim 4, wherein the input unit allows the user to input a selection instruction for selecting one item from the candidate list of the basic information, and wherein the processor is configured to retrieve additional information from the database in response to the selection instruction and provide the retrieved additional information to the display unit.

6. A method of providing patient information, comprising:
preparing database of patient information including basic information and additional information of patients;
receiving a user input in a syllable unit or a word unit from a user to produce at least one syllable or word;
retrieving basic information matched with the at least one syllable or word from the database; and
providing a candidate list of the basic information including the retrieved basic information.

7. The method of Claim 6, wherein the basic information includes ID, name and date of birth of the patient, and wherein the additional information includes medical departments, physical conditions, disease record, medical record, a doctor and patient's photo.

8. The method of Claim 7, further comprising:
a) receiving a first syllable or word of the basic information through an input unit from a user;
b) retrieving basic information including the received first syllable or word from the database;
c) forming and displaying a first candidate list of the basic information based on the retrieved basic information;
d) checking whether a second syllable or word of the basic information is inputted or whether a selection instruction for selecting one item from the first candidate list of the basic information through the input unit;
e) if it is determined that the second syllable or word is inputted at step d), retrieving basic information including the second syllable or word from the first candidate list of the basic information and forming and displaying a second candidate list of the basic information based on the retrieved basic information; and
f) if it is determined that the selection information is inputted at step d), retrieving additional information corresponding to the selected basic information and for an additional information providing window for display.

9. The method of Claim 8, wherein the step a) includes receiving the first syllable or word of one of ID, name and data of birth.
